**Europäisches Patentamt**

**European Patent Office**

**Office européen des brevets**

(19)

(11) Numéro de publication : **0 305 255 B1**

## (12) FASCICULE DE BREVET EUROPEEN

(45) Date de publication du fascicule du brevet :
**26.02.92 Bulletin 92/09**

(51) Int. Cl.⁵ : **A61F 2/16**

(21) Numéro de dépôt : **88402018.1**

(22) Date de dépôt : **03.08.88**

(54) **Lentille intraoculaire de chambre postérieure.**

(30) Priorité : **05.08.87 FR 8711340**

(43) Date de publication de la demande :
**01.03.89 Bulletin 89/09**

(45) Mention de la délivrance du brevet :
**26.02.92 Bulletin 92/09**

(84) Etats contractants désignés :
**AT BE CH DE ES FR GB GR IT LI LU NL SE**

(56) Documents cités :
**DE-A- 3 140 465**
**GB-A- 2 114 315**
**NL-A- 8 500 527**
**US-A- 4 280 232**
**US-A- 4 412 359**
**US-A- 4 477 931**

(73) Titulaire : **LABORATOIRES DOMILENS SOCIETE ANONYME**
**321 avenue Jean Jaurès**
**F-69007 Lyon (FR)**

(72) Inventeur : **Charleux, Jacques**
**63 rue Crillon**
**F-69006 Lyon (FR)**

(74) Mandataire : **Maureau, Philippe et al**
**Cabinet Germain & Maureau Le Britannia - Tour C 20, bld Eugène Déruelle Boîte Postale 3011**
**F-69392 Lyon Cédex 03 (FR)**

## Description

La présente invention a pour objet un implant intraoculaire de chambre postérieure de type intracapsulaire, c'est-à-dire un implant destiné à être mis en place dans la chambre postérieure de l'oeil, à l'intérieur de la capsule du cristallin lors d'une ablation de celui-ci à la suite d'une cataracte.

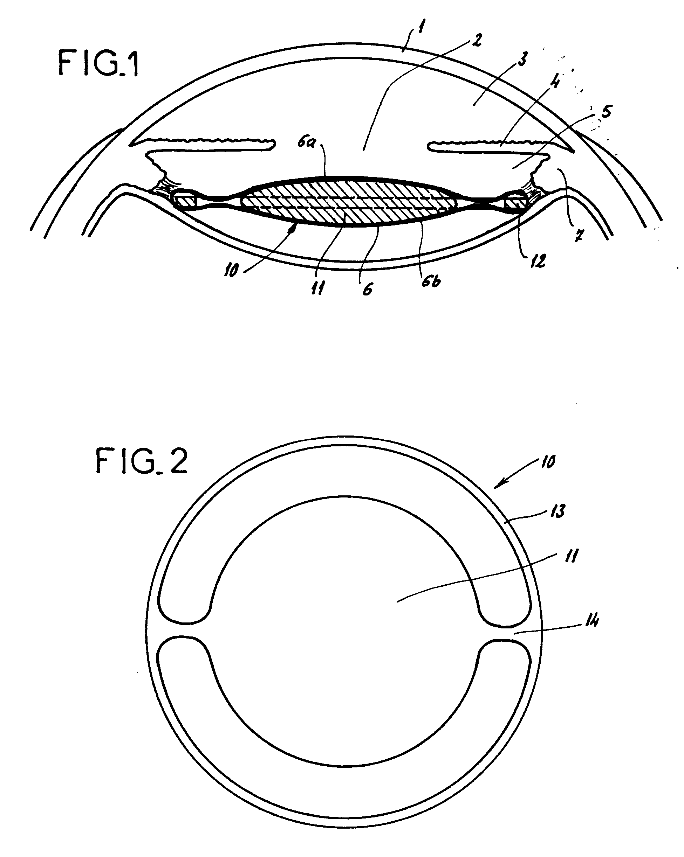

La figure 1 représente, en coupe transversale, la partie antérieure d'un globe oculaire à l'intérieur duquel est placé un implant 10 de ce type.

Ainsi que le montre cette figure, la chambre postérieure 5 est limitée à l'avant par l'iris 4 et à l'arrière par le corps ciliaire 7 et le cristallin 6.

La partie antérieure de l'oeil comprend en outre la cornée 1, la pupille 2 et l'iris 4 délimitant entre eux la chambre antérieure 3.

Comme indiqué précédemment, l'implant 10 de chambre postérieure intracapsulaire est mis en place à l'intérieur de la capsule, c'est-à-dire de l'enveloppe 6 du cristallin, ce dernier étant enlevé.

L'implant 10 de type connu, représenté en coupe transversale sur la figure 1, et en plan sur la figure 2, est constitué par une partie centrale 11 formant la lentille proprement dite et une partie d'appui 12.

La partie centrale 11 est biconvexe (cf figure 2) et la partie d'appui est constituée par une couronne circulaire 13 coaxiale à la partie centrale 11 et reliée à celle-ci par deux "ponts" 14 situés selon un même diamètre.

La forme circulaire de la partie d'appui 12 de cet implant permet un bon centrage de celui-ci dans la chambre postérieure de l'oeil.

Par ailleurs, l'intervalle de forme annulaire délimité entre la couronne d'appui 13 et la partie centrale 11 de l'implant permet une meilleure fixation de celui-ci dans la capsule du cristallin, par "soudure" entre elles des deux parois 6a, 6b de cette capsule.

Cependant cet implant présente certains inconvénients.

En effet la présence des deux ponts diamétraux lui confère une certaine rigidité ne facilitant pas sa mise en place.

Par ailleurs sa forme biconvexe favorise l'adhérence sur ses deux faces des parois de la capsule du cristallin. De ce fait, en cas de cataracte secondaire, c'est-à-dire de croissance de cellules opacifiant la capsule du cristallin après ablation de celui-ci, le rayonnement laser utilisé pour détruire ces cellules endommage également la surface de la lentille sur laquelle celles-ci sont collées.

Conformément au document GB-A-2 114 315, et plus précisément à sa figure 19, on a décrit un implant intraoculaire constitué par une partie centrale circulaire formant lentille, et par une partie d'appui en forme de couronne circulaire fermée, sur laquelle la partie centrale est suspendue par des fils.

Un tel implant est de réalisation difficile, en raison du caractère distinct de la partie centrale et de la partie d'appui, et de la suspension de la première sur la seconde. La manipulation d'un tel implant par le chirurgien apparaît difficile et aléatoire, et on n'est pas du tout assuré que l'implant retrouve sa forme d'origine, une fois mis en place dans l'oeil du patient.

Conformément au document US-C-4 477 931, on a décrit un implant intraoculaire de chambre postérieure, de type intracapsulaire, constitué par une partie centrale (21) formant lentille et par une partie d'appui (22) élastique constituée par deux anses reliées à la partie centrale par un pont.

En pratique, les deux anses souples et relativement libres de la partie d'appui n'ont pas à la fois une élasticité et une rigidité suffisante pour permettre un positionnement précis et fiable de l'implant dans le sac intracapsulaire.

Le but de la présente invention est de remédier à ces inconvénients et de concevoir un implant qui présente une grande souplesse, tout en se mettant bien en place à l'intérieur de la capsule du cristallin. La présente invention a aussi pour objet de résoudre de façon satisfaisante les problèmes liés à une cataracte secondaire.

Conformément à la présente invention, la partie d'appui est reliée à la partie centrale par une seule attache, formée en une seule pièce avec la partie d'appui et la partie centrale, et présentant une largeur relativement importante par rapport à celle de la partie d'appui ; et l'attache est inclinée par rapport à la partie centrale.

Cette configuration confère à l'implant une souplesse maximale permettant de le "replier" largement dans l'axe de l'attache pour son introduction dans la capsule du cristallin, et permettant donc de réduire l'incision nécessaire à son introduction. Une fois introduit dans la capsule du cristallin, l'implant, grâce à son élasticité, se détend et retrouve sa forme circulaire, pour venir se loger correctement dans la chambre postérieure de l'oeil .

Le centrage et la stabilité de l'implant ainsi obtenus sont excellents, car l'anse circulaire fermée permet un appui sur 360 ° à l'intérieur de l'oeil.

Le fait que l'attache soit inclinée par rapport à la partie centrale permet de "tendre" la membrane de la capsule sur l'implant, et donc d'éviter toute adhérence de celle-ci sur la partie centrale de celui-ci.

Selon une forme de réalisation avantageuse la partie centrale de l'implant est en forme de ménisque, ce qui permet d'éviter l'adhérence de la membrane de la capsule du cristallin sur cette partie centrale, notamment dans la partie concave de celle-ci. De ce fait, en cas de cataracte secondaire, les cellules opacifiant la membrane de la capsule peuvent être détruites par un rayonnement laser sans endommager la lentille.

De toute façon, l'invention sera mieux comprise, et d'autres caractéristiques de celle-ci seront mises en évidence à l'aide de la description qui suit en référence au dessin schématique annexé en illustrant une forme de réalisation préférée :

Figure 3 est une vue similaire à figure 1 montrant l'implant selon l'invention en place dans la capsule du cristallin.

Figure 4 est une vue en plan de l'implant selon l'invention,

Figure 5 est une vue en coupe selon V-V de figure 4.

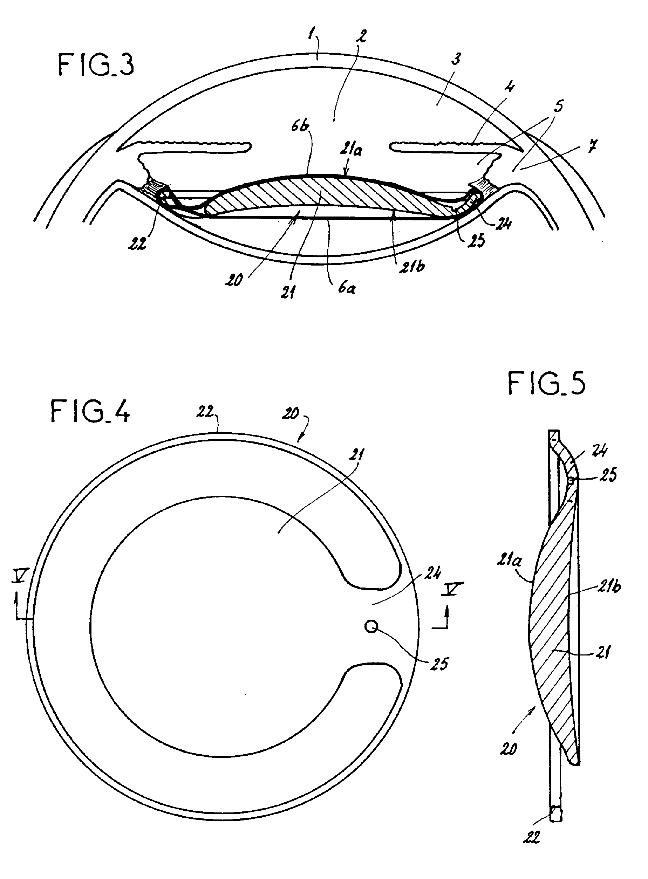

La figure 3 montre un implant 20 selon l'invention mis en place à l'intérieur de la capsule 6 du cristallin.

Ainsi que le montrent cette figure et la figure 4, cet implant 20 est constitué d'une partie centrale 21 de forme circulaire et constituant la lentille proprement dite et d'une partie d'appui 22 élastique.

La partie d'appui 22 est, de même que pour l'implant connu 10, en forme de couronne circulaire.

Cependant, à la différence de cet implant connu, elle n'est rattachée à la partie centrale 21 que par une seule attache 24. Cette caractéristique confère donc à l'implant une grande souplesse et permet de "replier" celui-ci au maximum lors de son introduction dans l'oeil.

Cette forme permet donc de réduire au maximum les incisions devant être pratiquées dans la cornée et dans la capsule pour la mise en place de l'implant.

Ainsi que le montre la comparaison des figures 2 et 4, l'attache 24 de l'implant selon l'invention est beaucoup plus large (environ dix fois plus large) que chacune des attaches 14 de l'implant connu et, de ce fait, l'implant selon l'invention est beaucoup moins fragile.

Dans cette forme préférée de réalisation, l'attache 24 présente une largeur environ dix fois supérieure à celle de la partie d'appui 22, ce qui constitue une garantie de sa solidité.

Par ailleurs, un trou non débouchant 25 est ménagé dans la partie centrale de cette attache 24 ; ce trou est destiné à recevoir l'extrémité d'un outil de type crochet permettant un positionnement correct de l'implant dans l'oeil par le chirurgien.

Ainsi que le montre la figure 5, la partie centrale 21 de l'implant est en forme de ménisque, c'est-à-dire qu'elle présente à la fois une paroi 21a convexe et une paroi 21b concave.

Par ailleurs, l'attache 24 est inclinée par rapport à la partie centrale.

L'angle $\alpha$, que forme cette attache 24 avec le plan général (P) de la lentille, est égal à environ 35°. Cette inclinaison de l'attache 24 par rapport à la partie centrale 21 présente plusieurs avantages : d'une part, lorsque l'implant est replié pour son introduction dans l'oeil, cette inclinaison ainsi que la courbure de la partie centrale, confèrent à l'implant une forme de capsule facilitant son glissement à l'intérieur de l'oeil et le soulèvement de la membrane constituant la capsule 6 du cristallin.

Cette inclinaison de l'attache 24 permet, d'autre part, de tendre la membrane constituant la capsule 6 du cristallin et notamment, ainsi que le montre la figure 3, de tendre la membrane intérieure 6a de celle-ci. De cette façon, la face concave 21b de l'implant se trouve séparée de la membrane intérieure 6a de la capsule et il n'y a donc aucun contact entre celles-ci.

Cette disposition est avantageuse en cas de cataracte secondaire, c'est-à-dire d'opacification de la membrane de la capsule 6 du cristallin.

En effet, dans un tel cas, les cellules opacifiant la membrane peuvent être détruites au moyen d'un rayonnement laser, sans qu'il n'y ait aucun risque d'endommagement de la paroi 21b de l'implant, du fait de l'absence de contact entre celui-ci et la membrane de la capsule du cristallin.

On notera, par ailleurs, que la forme circulaire de l'implant selon l'invention permet un bon centrage à l'intérieur de l'oeil et que la forme de couronne de sa partie d'appui permet une meilleure fixation de celui-ci à l'intérieur de l'oeil par "soudure" entre elles des deux membranes 6a,6b de la capsule du cristallin dans les intervalles existant entre la partie centrale 21 et la partie d'appui 22 de l'implant.

Comme il va de soi, la présente invention ne se limite pas à la seule forme d'exécution décrite ci-avant à titre d'exemple non limitatif.

C'est ainsi que, conformément à une variante de réalisation, la partie centrale formant lentille peut présenter une forme biconvexe de manière à la rapprocher de la forme du cristallin naturel.

Avantageusement, l'angle d'inclinaison de l'attache 24 par rapport à la partie centrale de l'implant est d'environ 20°.

Par ailleurs, le rapport du rayon de la face avant sur le rayon de la face arrière de cette partie centrale est d'environ 1/2, ce qui est très proche de la géométrie naturelle du cristallin.

## Revendications

1. Implant intraoculaire de chambre postérieure, de type intracapsulaire, constitué d'une partie centrale (21) formant lentille et d'une partie d'appui (22) élastique en forme de couronne circulaire fermée, **caractérisé en ce que** la partie d'appui (22) est reliée à la partie centrale (21) par une seule attache (24), formée en une seule pièce avec la partie d'appui (22) et la partie centrale (21), et présentant une largeur relativement importante par rapport à celle de la partie d'appui, et en ce que l'attache (24) est inclinée par rapport à la partie centrale (21).

2. Implant selon la revendication 1, caractérisé en ce que la largeur de l'attache (24) est environ dix fois supérieure à celle de la partie d'appui (22).

3. Implant selon la revendication 1 ou la revendication 2, caractérisé en ce que l'attache (24) est munie d'un trou non débouchant (25).

4. Implant selon l'une quelconque des revendications 1 à 3, caractérisé en ce que l'angle d'inclinaison de l'attache (24) par rapport à la partie centrale (21) est d'environ 35 °.

5. Implant selon l'une quelconque des revendications 1 à 4, caractérisé en ce que la partie centrale (21) est en forme de ménisque.

6. Implant selon l'une des revendications 1 à 3, caractérisé en ce que la partie centrale formant lentille présente une forme biconvexe.

7. Implant selon la revendication 6, caractérisé en ce que l'angle d'inclinaison de l'attache (24) par rapport à la partie centrale (21) est d'environ 20 °.

8. Implant selon la revendication 6 ou la revendication 7, caractérisé en ce que le rapport du rayon de la face avant sur le rayon de la face arrière de cette partie centrale est d'environ 1/2.

## Patentansprüche

1. Intraokulares intrakapsuläres Implantat für die hintere Augenkammer, bestehend aus einem eine Linse bildenden mittigen Abschnitt (21) und einem elastischen Abstützabschnitt (22) in Form eines geschlossenen kreisförmigen Kranzes, dadurch gekennzeichnet, daß der Abstützabschnitt (22) mit dem mittigen Abschnitt (21) über ein einziges Verbindungsstück (24) verbunden ist, das einstückig mit dem Abstützabschnitt (22) und dem mittigen Abschnitt (21) ist, und das relativ breit verglichen mit dem Abstützabschnitt ist, und daß das Verbindungsstück (24) hinsichtlich des mittigen Abschnitts (21) geneigt verläuft.

2. Implantat nach Anspruch 1, dadurch gekennzeichnet, daß die Breite des Verbindungsstückes (24) etwa zehn mal größer ist als die des Abstützabschnittes (22).

3. Implantat nach Anspruch 1 oder nach Anspruch 2, dadurch gekennzeichnet, daß im Verbindungsstück (24) eine Sacklochöffnung (25) ausgespart ist.

4. Implantat nach einem der Ansprüche 1 bis 3, dadurch gekennzeichnet, daß der Neigungswinkel des Verbindungsstückes (24) hinsichtlich des mittigen Abschnittes (21) etwa 35° beträgt.

5. Implantat nach einem der Ansprüche 1 bis 4, dadurch gekennzeichnet, daß der mittige Abschnitt (21) die Form eines Meniskus aufweist.

6. Implantat nach einem der Ansprüche 1 bis 3, dadurch gekennzeichnet, daß der mittige Abschnitt, der die Linse bildet, eine bikonvexe Form ausweist.

7. Implantat nach Anspruch 6, dadurch gekennzeichnet, daß der Neigungswinkel des Verbindungsstückes (24) im Hinblick auf den mittigen Abschnitt (21) etwa 20° beträgt.

8. Implantat nach Anspruch 6 oder nach Anspruch 7, dadurch gekennzeichnet, daß das Verhältnis zwischen dem Radius der vorderen Oberfläche und dem Radius der hinteren Oberfläche des mittigen Abschnitts etwa bei 0,5 liegt.

## Claims

1. Intraocular implant for the posterior chamber, of the intracapsular type, consisting of a central portion (21) forming a lens and an elastic supporting portion (22) having the shape of a closed circular ring, characterised in that the supporting portion (22) is joined to the central portion (21) by a single holding link (24), formed integrally with the supporting portion (22) and the central portion (21) and possessing a relatively large width compared to that of the supporting portion, and in that the holding link (24) is inclined with respect to the central portion (21).

2. Implant according to Claim 1, characterised in that the width of the holding link (24) is approximately ten times as great as that of the supporting portion (22).

3. Implant according to Claim 1 or Claim 2, characterised in that the holding link (24) is equipped with a blind hole (25).

4. Implant according to any one of Claims 1 to 3, characterised in that the angle of inclination of the holding link (24) with respect to the central portion (21) is approximately 35°.

5. Implant according to any one of Claims 1 to 4, characteriaed in that the central portion (21) is meniscus-shaped.

6. Implant according to one of Claims 1 to 3, characteriasd in that the central portion forming a lens possesses a biconvex shape.

7. Implant according to Claim 6, characteriaed in that the angle of inclination of the holding link (24) with respect to the central portion (21) is approximately 20°.

8. Implant according to Claim 6 or Claim 7, characterised in that the ratio of the radius of the front side to the radius of the rear side of this central portion is approximately 1:2.

FIG.1

FIG.2

FIG.3

FIG.4

FIG.5